# EUROPEAN PATENT SPECIFICATION

(11) **EP 3 608 396 B1**
(45) Date of publication and mention of the grant of the patent: **27.10.2021**
(21) Application number: 18774447.9
(22) Date of filing: 21.03.2018
(51) Int. Cl.: C12N 1/12, C02F 3/32, B01D 59/36, C22B 3/18, C22B 60/02, G21F 9/18, C12R 1/89

(54) **MICROORGANISM OF THE SPECIES TETRASELMIS MEDITERRANEA (TMMRU) AND USE THEREOF FOR THE PRODUCTION OF ENRICHED URANIUM**
MIKROORGANISMUS DER SPEZIES TETRASELMIS MEDITERRANEA (TMMRU) UND VERWENDUNG DAVON ZUR HERSTELLUNG VON ANGEREICHERTEM URAN
MICRO-ORGANISME DE L'ESPÈCE TETRASELMIS MEDITERRÁNEA (SOUCHE TMMRU) ET SON UTILISATION POUR LA PRODUCTION D'URANIUM ENRICHI

(30) Priority: 29.03.2017 ES 201700306
(43) Date of publication of application: 12.02.2020
(73) Proprietor: Universidad Complutense De Madrid, 28040 Madrid (ES)
(72) Inventor: LOPEZ RODAS, Victoria, 28040 Madrid (ES); COSTAS COSTAS, Eduardo, 28040 Madrid (ES); GARCIA BALBOA, Camino, 28040 Madrid (ES); ROMERO LOPEZ, Julia, 28040 Madrid (ES)
(74) Representative: Ungria López, Javier
(86) International application number: PCT/ES2018/000024
(87) International publication number: WO 2018/178416

(56) References cited:
- WO-A1-2014/160350
- WO-A2-2004/038491
- ES-A1- 2 531 010
- GB-A- 1 472 626
- GB-A- 1 507 003
- US-A1- 2008 268 513
- US-A1- 2013 078 707
- US-A1- 2015 004 674

## Description

### Technical field of the invention

The present invention falls within the sector of Biotechnology applied to the Energy sector and, in particular, would be of use in Uranium Enrichment Plants and in radioactive waste storage plants for the reprocessing of depleted uranium. The invention therefore also relates to the environmental sector.

### Description of the state of the art

Although nuclear energy is one of the energy sources that contributes least to environmental problems such as global warming or acid rain, the generation of radioactive waste, unforeseen nuclear accidents and the association that many people tend to make between nuclear energy and "atomic bombs" have led the public opinion in general to adopt a position of rejection towards any uranium-related process. However, despite the unfavorable opinions, nuclear energy is currently one of the main sources of power generation in the world: for example, in the United States it represents 20% of the electricity produced and countries such as France, United Kingdom and Russia have 57, 33 and 30 nuclear reactors, respectively (Czerwinski K., Polz M., 2008. Uranium enrichment using microorganisms. Patent No. US 2008/028513 A1).

The public image of nuclear technology would undoubtedly improve and would benefit from the viewpoint of efficiency if the environmental impact of any of the uranium life cycle stages (extraction, processing and enrichment) could be reduced. More conservative processes from an energy viewpoint and procedures that generate less waste would be desirable objectives.

Uranium enrichment plants are the primary target of social rejection. The starting point to change the current vision of isotopic fractionation as a harmful activity would be to explain that isotopic fractionation processes are not so far removed from natural processes. Indeed, isotopic fractionation is a physicochemical process that may occur in natural situations wherein separation is possible depending on mass. For example, clouds are enriched in the lightest oxygen isotope (¹⁶O).

Isotopic fractionation also occurs in most biological processes, although its effects are normally negligible, with the exception of some cases related to hydrogen or deuterium (Bowen HJM, 1960, Biological fractionation of isotopes. International Journal of Applied Radiation and Isotopes, vol. 7. pp. 261-272). This natural fractionation occurs as a consequence of the differences in the speed of diffusion of the different isotopes: in general, lighter isotopes travel faster than heavier isotopes via metabolic pathways. For example, in photosynthetic processes, the CO₂ captured is enriched in ¹²C while the oxygen released is enriched in ¹⁸O. The differences in the isotopic composition of sulfur, nitrogen and carbon used to trace food chains (Hoefs J., Stable Isotope Geochemistry. Springer-Verlag, Berlin, 1997, 4th ed.) or the different ratios C¹²/C¹³ to determine the biogenic origin of old rocks are closest.

Although the natural capacity of some microorganisms and microbial biomass to recover uranium has been studied, the possibility of enriching uranium using new methods based on microbial activity has scarcely been proposed, despite the existence of a series of encouraging indicators. Isotopic fractionation has been observed in reducing bacteria, for example sulfur fractionation during the dissimilatory reduction of sulfates (Johnson TM, 2004: A review of mass-dependent fractionation of selenium isotopes and implications for other heavy stable isotopes. Chem Geol 204: 201-214); the isotopic fractionation of Fe at the time of dissimilatory reduction of Fe(III) (Crosby HA., Roden EE., Johnson CE., Beard BL., 2007). The mechanisms of iron isotope fractionation during dissimilatory Fe(III) reduction by Shewanella putrefaciens and Geobacter sulfurreducens. Geobiology 5: 169-189) and dissimilatory reduction of selenium (Johnson, 2004). The effectiveness of actinide-reducing organisms to separate the heaviest isotope by precipitation has also been assayed (Basu A., 2013. Isotopic fractionation of chromium and uranium during abiotic and microbial Cr(VI) reduction and microbial U(VI) reduction, Doctoral Thesis, 2103).

The estimated amount of depleted uranium (the so-called "tailings" of the enrichment process in the ²³⁵U isotope) accumulated in the world is estimated at 1.5 million tonnes. Given that the average isotopic abundance or concentration of ²³⁵U therein is 0.25% (compared to the natural concentration of 0.711%), it can be deducted that there is still 35% of ²³⁵U (fissile isotope) stored for future use should there be a cost-effective extraction process (isotopic enrichment). "Traditional" physical uranium enrichment processes do not apply (are not efficient) when the concentration is very low, therefore extracting ²³⁵U from those tailings is not currently cost-effective.

The present invention proposes a technological development based on micro-algae technology, which enables uranium tailings enrichment. Specifically, the use of a marine micro-algae is proposed, *Tetraselmis mediterranea TmmRU,* which was isolated from a marine medium and subjected to genetic improvement by artificial selection until obtaining the most effective clone in uranium fractionation. The microalgae strain *Tetraselmis mediterranea TmmRU* selectively captures ²³⁵U and is capable of enriching "tailings", considered "waste", with ²³⁵U effectively and at a reasonable cost.

### Object of the Invention

The invention relates to a new microalgae strain, *Tetraselmis mediterranea TmmRU,* which is capable of selectively capturing ²³⁵U, which converts it into a very useful species for the isotopic enrichment (isotopic fractionation) of uranium.

Therefore, the present invention provides the strain *Tetraselmis mediterranea TmmRU,* which was subjected to an artificial genetic improvement process to obtain a strain with high capacity to selectively capture the ²³⁵U isotope, giving rise to an enrichment in ²³⁵U with respect to the ratio present in a starting solution.

Therefore, the first aspect of the present invention relates to a microorganism of the micro-algae species *Tetraselmis mediterranea TmmRU* with Spanish Algae Bank access number BEA/IDA/0062.

Another aspect of the present invention relates to the use of a microorganism of the species *Tetraselmis mediterranea TmmRU* to increase the ratio ²³⁵U/²³⁸U in liquid solutions in relation to a starting solution. In a preferred embodiment of this aspect of the invention, the microorganism belonging to the micro-algae species *Tetraselmis mediterranea TmmRU* is the microorganism of the invention.

In the present invention, "enriched uranium" is understood to be a solution wherein the ratio ²³⁵U/²³⁸U has been increased by a certain factor of the isotopic ratio of a starting solution.

Another aspect of the present invention relates to a method for obtaining solutions enriched in ²³⁵U using the microorganism of the invention, comprising the following steps:
a. Culturing a microorganism of the species *Tetraselmis mediterranea TmmRU,* preferably the microorganism of the invention
b. Separating the algal biomass produced by the microorganism of the invention
c. Recovering the uranium from the algal biomass through an acid digestion process of said biomass.

In step a. of the method of the invention, the culture of the microorganism is carried out in a growth medium and under conditions that favor the growth of said microorganism.

Thus, preferably, the culture conditions of this step of the method of the invention are: 80 µmol photons m⁻².s⁻¹ of light; 22°C of temperature and f/2 culture medium.

The most important novelty of the invention is that it uses a biotechnological method whereby enrichment occurs as a consequence of the specific selection of ²³⁵U by the algae, which implies the selective capture of the ²³⁵U isotope by the algae and not as a consequence of any indirect chemical reaction.

That is, the process through which the selective separation of the fissionable isotope (²³⁵U) is achieved differs from any of those currently used: toxic reagents (hexafluoride) or a system that consumes energy (centrifuges) are not used. In the only applications described in which microorganisms are used, the essence of the process is based on the use of indirect or collateral resources. In the case proposed herein, the selective isotopic separation process is carried out by the alga that captures the isotope ²³⁵U.

The system is highly versatile in its implementation. Bioreactors or even plant cooling ponds could be used.

The method is directly applicable to the use of wastewater containing "depleted" uranium, commonly called "uranium tailings".

### Description of the invention

In order to carry out enrichment in ²³⁵U of effluents containing "depleted" uranium (solutions with an isotopic ratio ²³⁵U/²³⁸U in the order of 0.2%), a reactor containing culture medium specific to the growth of marine micro-algae species will be prepared. Here the effluent that is the subject of the enrichment will be added.

Subsequently, the micro-algae species will be inoculated in a concentration of the order of 100,000 cells/ml. The culture can be maintained at room temperature and with a direct light source. Ideally, the culture can be thermostatized at 22°C and illuminated by a direct light source in the order of 80 µmol photons m⁻².s⁻¹.

After 20 days, the biomass will be separated from the solution by means of a unit operation which can be centrifugation or filtration. The biomass containing enriched uranium will be used as an inoculum in a new reactor, repeating the process as often as necessary in accordance with the desired enrichment. For example, a desirable objective is, starting with effluents with enrichment in ²³⁵U/²³⁸U in the order of 0.2%, to achieve as near as possible to 0.7% in ²³⁵U/²³⁸U.

Lastly, the recovery of enriched uranium contained in the biomass is carried out by means of acid digestion.

### Description of the figure

In order to facilitate the description herein and for the purpose of helping to make the invention more readily understandable, in accordance with a preferred practical embodiment thereof, said description is accompanied by a drawing constituting an integral part thereof which, by way of illustration and not limitation, represents a schematic diagram explaining the method that must be carried out for the isotopic uranium enrichment process that is the subject of the present invention.

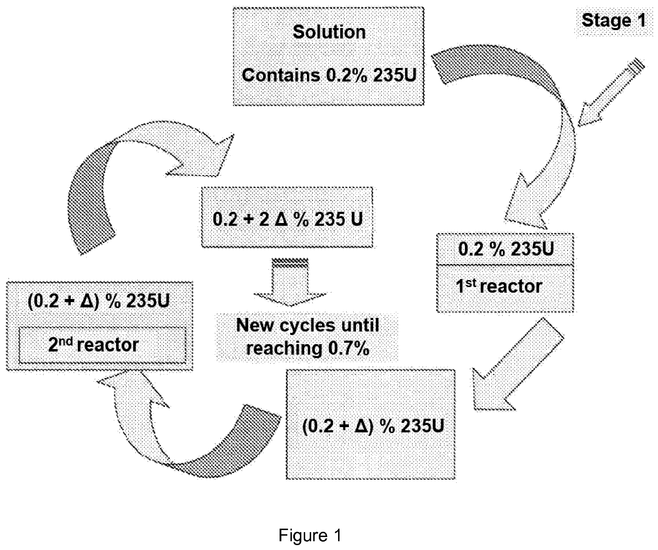

Figure 1 shows how, starting from wastewater with an isotopic ratio ²³⁵U/²³⁸U in the order of 0.2%, an isotopic ratio in the order of 0.7% can be achieved through successive enrichment stages. Figure 1 shows an inoculum of the micro-algae species *Tetraselmis mediterranea TmmRU* being transferred to the wastewater containing the depleted uranium (stage 1). The culture is supplemented with a nutrient medium to favor algal growth. After a period of approximately 20 days, the biomass can be separated from the aqueous medium that is enriched in a certain factor Δ. The microalgae biomass enriched in ²³⁵U must be separated in a subsequent stage through a filtration process, for example, and will be used to start a new cycle.

### Detailed description of at least one embodiment of the invention

The first part of the method consists of preparing a microalgae inoculum of the species *Tetraselmis mediterranea TmmRU.* This species must be grown in a uranium-containing medium until obtaining a cell density of at least 5 × 10⁶ cells/ml. Once obtained, an inoculum thereof will be transferred to the aqueous medium containing uranium dissolved in an isotopic ratio ²³⁵U/²³⁸U in the order of 0.2-0.3%. Additionally, 10% (vol/vol) of a nutrient medium will be added. The culture will be maintained at 20-25°C and under light. After 20 days, the biomass will be decanted and subsequently centrifuged or filtered, and transferred again to a subsequent stage wherein a new enrichment value Δ will be achieved (figure 1). Centrifugation is carried out preferably at 3,500 r.p.m. for 10 minutes; if filtration is chosen, it must be carried out in a vacuum using a filter with a pore size of 0.22 µm. The uranium contained in the biomass will be recovered by carrying out a drying stage up to a constant weight, followed by treatment with 2% v/v nitric acid.

### Practical example

A culture of *Tetraselmis mediterranea TmmRU* in f/2 medium (specific to marine microalgae growth) was prepared. Said culture was maintained with 80 µmol photons m⁻².s⁻¹ of light and at 22°C. Next, a solution of 2 mg/l uranium was inoculated, wherein the starting uranium isotopic ratio was 0.2%. This solution also contained the nutrient medium. The size of the inoculum was 100,000 cells/ml.

After 20 days, the biomass of the solution was separated by means of a centrifugation process at 3,500 r.p.m. for 10 minutes. Next, acid digestion was carried out followed by an evaporation to dryness stage. Lastly, the isotopic ratio for the uranium isotopes ²³⁵U and ²³⁸U was measured in both the biomass and the effluent, finding that it had achieved an enrichment in ²³⁵U in the biomass with respect to the liquid in a factor of 0.404833315.

## Claims

1. A micro-algae microorganism *Tetraselmis mediterranea TmmRU* with Spanish Algae Bank identification number BEA/IDA/0062.

2. Use of the microorganism, according to claim 1, in a method for the production of a uranium solution with a ratio ²³⁵U/²³⁸U enriched in ²³⁵U in relation to a starting solution, i.e. a uranium solution containing a ratio ²³⁵U/²³⁸U greater than the ratio ²³⁵U/²³⁸U in the starting solution.

3. Use, according to claim 2, for the production of a solution with an isotopic ratio ²³⁵U/²³⁸U of 0.7%.

4. A method for the production of a solution enriched in ²³⁵U with respect to the starting solution comprising the following stages:
a. Culturing the microorganism, according to claim 1, in a uranium medium also containing an adequate culture medium and under temperature conditions between 18-28°C and light conditions between 60-100 µmol photons/m²s
b. Separating the algal biomass obtained according to step a. by means of a centrifugation or filtration stage.
c. Obtaining a solution enriched in ²³⁵U with respect to the starting solution from the algal biomass obtained in stage b. by means of an acid digestion process.

5. The method, according to the preceding claims, **characterized in that** the uranium solution of stage a has an isotopic ratio ²³⁵U/²³⁸U of 0.2%.

6. The method, according to the preceding claims, **characterized in that** the size of the inoculum of the alga *Tetraselmis mediterranea TmmR* used in stage a ranges between 50,000 cells/ml-150,000 cells/ml.

7. The method, according to the preceding claims, **characterized in that** the size of the inoculum of the alga *Tetraselmis mediterranea TmmRU* used in stage a is 100,000 cells/ml.

8. The method, according to the preceding claims, **characterized in that** the culture medium is f/2 medium.

9. The method, according to the preceding claims, **characterized in that** the temperature and light conditions of stage a are carried out at 22°C and 80 µmol photons/m²s.

10. The method, according to claim 4, **characterized in that** the centrifugation of stage b is carried out at 3,500 r.p.m. for 10 minutes.

11. The method, according to claim 4, **characterized in** the filtration of stage b. is carried out under vacuum conditions using a filter with a pore size of 22 µm.

12. The method, according to claim 4, **characterized in that** the digestion of stage c is carried out in two stages: a drying stage up to a constant weight followed by treatment with 2% v/v nitric acid.

## Patentansprüche

1. Ein Mikroalgen-Mikroorganismus Tetraselmis mediterranea TmmRU mit der Identifikationsnummer BEA/IDA/0062 der spanischen Algenbank.

2. Verwendung des Mikroorganismus nach Anspruch 1 in einem Verfahren zur Herstellung einer Uranlösung mit einem in Bezug auf eine Ausgangslösung mit ²³⁵U angereicherten Verhältnis ²³⁵U/²³⁸U, d.h. einer Uranlösung, enthaltend ein Verhältnis ²³⁵U/²³⁸U, das größer ist als das Verhältnis ²³⁵U/²³⁸U in der Ausgangslösung.

3. Verwendung nach Anspruch 2 zur Herstellung einer Lösung mit einem Isotopenverhältnis ²³⁵U/²³⁸U von 0,7 %.

4. Verfahren zur Herstellung einer Lösung, die in Bezug auf die Ausgangslösung mit ²³⁵U angereichert ist, umfassend die folgenden Stufen:
a. Kultivieren des Mikroorganismus nach Anspruch 1 in einem Uranmedium, das auch ein geeignetes Kulturmedium enthält, unter Temperaturbedingungen zwischen 18-28°C und Lichtbedingungen zwischen 60-100 µmol Photonen/m²s
b. Abtrennen der gemäß Schritt a. gewonnenen Algenbiomasse durch eine Zentrifugations- oder Filtrationsstufe.
c. Gewinnen einer in Bezug auf die Ausgangslösung mit ²³⁵U angereicherten Lösung aus der in Sufe b. gewonnenen Algenbiomasse durch einen Säureaufschluss.

5. Verfahren nach den vorhergehenden Ansprüchen, **dadurch gekennzeichnet, dass** die Uranlösung der Stufe a ein Isotopenverhältnis ²³⁵U/²³⁸U von 0,2 % aufweist.

6. Verfahren nach den vorhergehenden Ansprüchen, **dadurch gekennzeichnet, dass** die Größe des in Stufe a verwendeten Inokulums der Alge Tetraselmis mediterranea TmmR zwischen 50.000 Zellen/ml und 150.000 Zellen/ml liegt.

7. Verfahren nach den vorhergehenden Ansprüchen, **dadurch gekennzeichnet, dass** die Größe des in Stufe a verwendeten Inokulums der Alge Tetraselmis mediterranea TmmRU 100.000 Zellen/ml beträgt.

8. Verfahren nach den vorhergehenden Ansprüchen, **dadurch gekennzeichnet, dass** das Kulturmedium f/2-Medium ist.

9. Verfahren nach einem der vorhergehenden Ansprüche, **dadurch gekennzeichnet, dass** die Temperatur- und Lichtbedingungen der Stufe a bei 22°C und 80 µmol Photonen/m²s durchgeführt werden.

10. Verfahren nach Anspruch 4, **dadurch gekennzeichnet, dass** die Zentrifugation der Stufe b bei 3.500 U/min für 10 Minuten durchgeführt wird.

11. Verfahren nach Anspruch 4, **dadurch gekennzeichnet, dass** die Filtration der Stufe b unter Vakuumbedingungen unter Verwendung eines Filters mit einer Porengröße von 22 µm durchgeführt wird.

12. Verfahren nach Anspruch 4, **dadurch gekennzeichnet, dass** der Aufschluss der Stufe c in zwei Stufen durchgeführt wird: einer Trocknungsstufe bis zu einem konstanten Gewicht, gefolgt von einer Behandlung mit 2% v/v Salpetersäure.

## Revendications

1. Microorganisme de l'espèce de micro-algue *Tetrase*/*mis mediterranea TmmRU* ayant le numéro d'identification de la Banque Espagnole d'Algues BEA/IDA/0062.

2. Utilisation du microorganisme, selon la revendication 1, dans un procédé pour la production d'une solution d'uranium ayant un rapport ²³⁵U/²³⁸U enrichi en ²³⁵U par rapport à une solution de départ, c'est-à-dire une solution d'uranium contenant un rapport ²³⁵U/²³⁸U supérieur au rapport ²³⁵U/²³⁸U dans la solution de départ.

3. Utilisation selon la revendication 2 pour la production d'une solution ayant un rapport des isotopes ²³⁵U/²³⁸U de 0,7 %.

4. Procédé pour la production d'une solution enrichie en ²³⁵U par rapport à la solution de départ, comprenant les étapes suivantes :
a. culture du microorganisme, selon la revendication 1, dans un milieu d'uranium contenant aussi un milieu de culture adéquat et dans des conditions de température comprise entre 18 et 28°C et des conditions de lumière comprise entre 60 et 100 µmol de photons/m²s,
b. séparation de la biomasse d'algue obtenue conformément à l'étape a. au moyen d'une étape de centrifugation ou de filtration,
c. obtention d'une solution enrichie en ²³⁵U par rapport à la solution de départ à partir de la biomasse d'algue obtenue dans l'étape b. au moyen d'un procédé de digestion par un acide.

5. Procédé selon les revendications précédentes, **caractérisé en ce que** la solution d'uranium de l'étape a a un rapport des isotopes ²³¹U/²³¹U de 0,2 %_{.}

6. Procédé selon les revendications précédentes, **caractérisé en ce que** la taille de l'inoculat de l'algue *Tetrase*/*mis mediterranea TmmR* utilisée dans l'étape a est située dans la plage comprise entre 50 000 cellules/ml et 150 000 cellules/ml.

7. Procédé selon les revendications précédentes, **caractérisé en ce que** la taille de l'inoculat de l'algue *Tetrase*/*mis mediterranea TmmRU* utilisée dans l'étape a est de 100 000 cellules/ml.

8. Procédé selon les revendications précédentes, **caractérisé en ce que** le milieu de culture est du milieu f/2.

9. Procédé selon les revendications précédentes, **caractérisé en ce que** les conditions de température et de lumière de l'étape a sont de 22°C et 80 µmol de photons/m²s.

10. Procédé selon la revendication 4, **caractérisé en ce que** la centrifugation de l'étape b est effectuée à 3 500 t/min pendant 10 minutes.

11. Procédé selon la revendication 4, **caractérisé en ce que** la filtration de l'étape b. est effectuée dans des conditions de vide par utilisation d'un filtre ayant une taille de pores de 22 µm.

12. Procédé selon la revendication 4, **caractérisé en ce que** la digestion de l'étape c est effectuée en deux étapes : une étape de séchage jusqu'à un poids constant, suivie d'un traitement avec de l'acide nitrique à 2 % v/v.
